(19) Europäisches Patentamt
European Patent Office
Office européen des brevets

(11) EP 2 726 453 B1

(12) **EUROPEAN PATENT SPECIFICATION**

(45) Date of publication and mention of the grant of the patent:
**03.06.2015 Bulletin 2015/23**

(21) Application number: **12737951.9**

(22) Date of filing: **29.06.2012**

(51) Int Cl.:
*C07C 291/02* (2006.01) *G03F 7/00* (2006.01)
*G03F 7/004* (2006.01) *G03H 1/02* (2006.01)

(86) International application number:
**PCT/US2012/044778**

(87) International publication number:
**WO 2013/003666 (03.01.2013 Gazette 2013/01)**

(54) **HOLOGRAPHIC RECORDING MEDIUM**

HOLOGRAFISCHES AUFZEICHNUNGSMEDIUM

SUPPORT D'ENREGISTREMENT HOLOGRAPHIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.06.2011 US 201113171640**
**20.10.2011 US 201113277659**

(43) Date of publication of application:
**07.05.2014 Bulletin 2014/19**

(73) Proprietor: **SABIC Innovative Plastics IP B.V.**
**4612 PX Bergen op Zoom (NL)**

(72) Inventors:
- **NAIK, Shantaram Narayan**
  **Bangalore**
  **Karnataka 560017 (IN)**
- **TAKEMORI, Michael T.**
  **Rexford, New York 12148 (US)**
- **JAIN, Sumeet**
  **Schenectady, New York 12308 (US)**
- **NADKARNI, Pradeep**
  **Bangalore**
  **Karnataka 560093 (IN)**
- **CHEVERTON, Mark A.**
  **Mechanicville, New York 12118 (US)**
- **PUTHAMANE, Kiran Arunkumar**
  **South Kodagu**
  **Karnataka 571216 (IN)**
- **VASUDEVAN, Vinodkumar**
  **Kerala 690106 (IN)**
- **DAVIS, Gary**
  **Albany, New York 12205 (US)**

(74) Representative: **Stiebe, Lars Magnus et al**
**Balder**
**Paseo de la Castellana 93**
**28046 Madrid (ES)**

(56) References cited:
**DE-A1-102008 047 409** **US-A1- 2009 081 560**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 726 453 B1

## Description

BACKGROUND

The present disclosure relates to media for recording and storing holograms, and more specifically to holographic recording media that include nitrone compounds useful as photoreactive active dyes.

Holograms are typically formed by interference fringes in a holographic recording medium that diffract light in a pattern to create a readable or viewable hologram. A variety of techniques have been used to create different types of holograms. Volume holograms are an increasingly popular mechanism for the authentication of genuine articles, whether it is for security purposes or for brand protection. Volume holograms can also be used for other purposes, such as data storage, or for decorative, illustrative, or artistic purposes. The use of volume holograms for authentication purposes is driven primarily by the relative difficulty with which they can be duplicated. Volume holograms are created by interfering two coherent beams of light to create an interference pattern and storing that pattern in a holographic recording medium. Information or imagery can be stored in a hologram by imparting the data or image to one of the two coherent beams prior to their interference. The hologram can be read out by illuminating it with a beam of light matching the geometry and wavelength of either of the two original beams used to create the hologram and any data or images stored in the hologram will be displayed. As a result of the complex methods required to record holograms, their use for authentication can be seen on articles such as credit cards, software, passports, clothing, and the like.

Early holographic recording media employed inorganic photorefractive crystals, such as doped or undoped lithium niobate ($LiNbO_3$), in which incident light creates refractive index changes. These index changes are due to the photo-induced creation and subsequent trapping of electrons leading to an induced internal electric field that ultimately modifies the index through a linear electro-optic effect. However, $LiNbO_3$ is expensive, exhibits relatively poor efficiency, fades over time, and requires thick crystals to observe any significant index changes.

More recent work has led to the development of polymers that can sustain larger refractive index changes owing to optically induced polymerization processes. These materials, which are referred to as photopolymers, have significantly improved optical sensitivity and efficiency relative to $LiNbO_3$ and its variants. Photopolymer holographic recording media (as disclosed in e.g., U.S. Patents US 7,824,822 B2, US 7,704,643 B2, US 4,996,120 A, US 5,013,632 A). "Single-chemistry" photopolymer systems have been employed, wherein the media comprise a homogeneous mixture of at least one photoactive polymerizable liquid monomer or oligomer, an initiator, an inert polymeric filler, and optionally a sensitizer. Since it initially has a large fraction of the mixture in monomeric or oligomeric form, the medium can have a gel-like consistency that necessitates an ultraviolet (UV) curing step to provide form and stability. Unfortunately, the UV curing step can consume a large portion of the photoactive monomer or oligomer, leaving significantly less photoactive monomer or oligomer available for data storage. Furthermore, even under highly controlled curing conditions, the UV curing step can often result in variable degrees of polymerization and, consequently, poor uniformity among media samples.

Other types of media and recording techniques have also been used to generate volume holograms. For example, dichromated gelatin, liquid crystal materials, photographic emulsions, and others as disclosed in P. Hariharan, Optical Holography - Principles, techniques, and applications 2nd ed., Cambridge University Press, 1996, can all be used to generate volume holograms.

Another type of holographic recording medium utilizes a photoreactive dye dispersed in a polymer binder. This approach offers a number of advantages. Unlike many other volume holographic recording media, photoreactive dye-based media can be used with a variety of different types of polymer binders such as thermoplastics that can provide a number of beneficial physical properties specific to particular class(es) of thermoplastics (e.g., polycarbonates), such as toughness, optical clarity, scratch resistance, strength, flexibility, and the like, as well as ease of fabricating holographic articles using conventional thermoplastic fabrication techniques. Different classes of thermoplastics have different combinations of advantages and disadvantages, which can be leveraged for the desired final application.

Photoreactive dyes are compounds that undergo a light induced chemical reaction when exposed to a wavelength within the absorption range of the dye to form at least one photoproduct. This reaction can be a photodecomposition reaction, such as oxidation, reduction, or bond breaking to form smaller constituents, or a molecular rearrangement, such as a sigmatropic rearrangement, or addition reactions including pericyclic cycloadditions. The light exposure-induced chemical reaction causes a difference in refractive index in the holographic recording medium between exposed and unexposed portions of the medium, which allows for light diffracting interference fringe patterns to be formed when the medium is exposed to mutually coherent interfering signal and reference light sources.

The performance of photoreactive dyes for use in holographic recording media can be evaluated by a variety of different criteria, including but not limited to quantum efficiency, diffraction efficiency, sensitivity, thermal stability, spectral absorbance, and compatibility with polymer binder(s) used in a holographic recording/storage medium.

Quantum efficiency (QE) is a measure of the probability of a photochemical transition for each absorbed photon of a given wavelength, and thus is a measure of efficiency with which incident light is used to achieve photochemical

conversion of the dye from one molecular form to another. Quantum efficiency is given by the equation

$$QE = \frac{hc/\lambda}{\sigma \cdot F_0}$$

where h is Planck's constant, c is the velocity of light, $\lambda$ is the wavelength of the light being absorbed, $\sigma$ is the absorption cross-section at the wavelength $\lambda$, and $F_0$ is the light fluence. The absorption cross-section is a measurement of the ability of an atom or molecule to absorb light at a specified wavelength, and is governed by the Beer-Lambert law for optically thin samples as shown in the equation

$$\sigma = \ln(10) \cdot \frac{Absorbance}{N_0 \cdot L}(cm^2)$$

where $N_0$ is the concentration in molecules per $cm^3$ and $L$ is the sample thickness in cm. Generally, higher QE values are beneficial for photoreactive dyes in holographic applications, as they potentially require lower (i.e., shorter and/or lower intensity) exposures to actinic radiation in order to produce a photochemically-induced change in the dye compound.

Diffraction efficiency (DE or $\eta$) is a measure of the difference in refractive index in the holographic recording/storage medium between exposed and unexposed portions, which is produced by the photoreactive dye in response to exposure. It is determined experimentally by measuring the diffracted power of the hologram and then calculating the diffraction efficiency, $\eta$, according to the equation

$$\eta = \frac{P_{diffracted}}{P_{reference}}$$

where $P_{diffracted}$ is the diffracted power of the hologram.

Sensitivity (S) is a measure of the diffraction efficiency of a hologram recorded using a certain amount of light fluence, F. Light fluence, F, is determined as the product of light intensity (I) and exposure time (t). Sensitivity can be represented by the equation

$$S = \frac{\sqrt{\eta}}{I \cdot t \cdot L}(cm/J)$$

where I is the intensity of the recording beam, *t* is the recording time, and *L* is the thickness of the holographic recording/storage medium, and $\eta$ is the diffraction efficiency.

Various compounds have been proposed as photoreactive dyes for holographic recording and storage. For example, see US 2006/0073392 A1, US 2009/0081560 A1, and US 2009/0082580 A1. However, there continues to be a need for new compounds for this purpose that provide beneficial properties, including but not limited to any or all of good quantum efficiency, diffraction efficiency, sensitivity, thermal stability, spectral absorbance, and/or compatibility with polymer binder(s) used in a holographic recording/storage medium.

SUMMARY

Disclosed herein is a holographic recording composition comprising a polymer binder and a nitrone compound according to the formula:

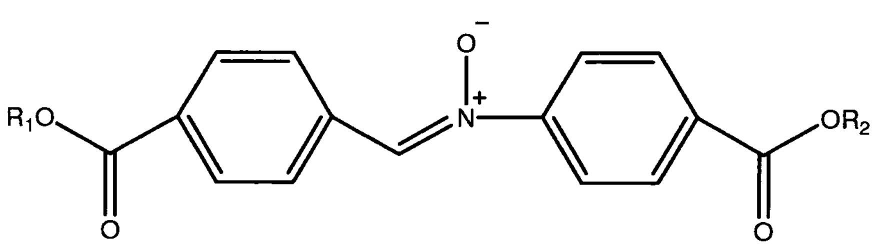

(I)

wherein $R_1$ and $R_2$ are each independently $C_1$-$C_{10}$ alkyl.

Disclosed herein too is an article comprising the nitrone photochromic dye of formula (I) and an organic material, wherein the article is used as a data storage media.

Disclosed herein in addition, is a method for recording information comprising exposing an article that comprises the nitrone photochromic dye of formula (I) to mutually coherent signal and reference light sources at a wavelength that causes a change in the chemical structure of the compound of formula (I).

Nitrones of formula (I), because of their high decomposition temperatures are suitable for use in thermoplastic compositions used in forming (e.g., by injection molding) photoactive components for holographic applications. Injection molding in holographic applications improves the processing of such holographic media and allows the use of polycarbonates and other high $T_g$ (glass transition temperature) thermoplastic resins.

DETAILED DESCRIPTION

As noted, holographic data storage relies upon the introduction of localized variations in the refractive index of the optically transparent substrate comprising the photoreactive dye as a means of storing holograms. The refractive index within an individual volume element of the optically transparent substrate can be constant throughout the volume element, as in the case of a volume element that has not been exposed to electromagnetic radiation, or in the case of a volume element in which the photoreactive dye has been reacted to the same degree throughout the volume element. It is believed that most volume elements that have been exposed to electromagnetic radiation during the holographic data writing process will contain a complex holographic pattern, and as such, the refractive index within the volume element will vary across the volume element. In instances in which the refractive index within the volume element varies across the volume element, it is convenient to regard the volume element as having an "average refractive index" which can be compared to the refractive index of the corresponding volume element before irradiation. Thus, in one embodiment an optically readable datum comprises at least one volume element having a refractive index that is different from a corresponding volume element of the optically transparent substrate before irradiation. Data storage is achieved by locally changing the refractive index of the data storage medium in a graded fashion (continuous sinusoidal variations), rather than discrete steps, and then using the induced changes as diffractive optical elements.

In one embodiment of the invention, a holographic recording medium comprising an optically transparent substrate is provided. The optically transparent substrate can be made of materials possessing sufficient optical quality such as, low scatter, low birefringence, and negligible losses at the wavelengths of interest, to render the data stored in the holographic storage medium readable. Generally, plastic materials that exhibit these properties can be used as the substrate. However, the plastic materials should be capable of withstanding the particular processing parameters employed (e.g., inclusion of the dye, exposure to a sensitizing solvent and application of any coating or subsequent layers, and molding it into a final format) and subsequent storage conditions.

Disclosed herein are novel nitrone compounds useful as photoreactive dyes and optical data storage media, containing the nitrone compounds, for use in holographic data storage and retrieval. Also disclosed are methods directed to holographic storage media preparation, data storage, and data retrieval. The holographic recording medium is manufactured from a holographic recording composition that comprises a binder composition and a photoactive material, wherein the photoactive material comprises a photochromic dye. The photochromic dye comprises a nitrone compound of formula (I). The holographic recording media can be advantageously used for data storage. The holographic recording media can also be written and read (i.e., data can be stored and retrieved respectively) using electromagnetic radiation having the same wavelength.

The nitrones of formula (I), due to their high decomposition temperatures, can be processed at temperatures needed for processing (e.g., molding and extruding) for a variety of thermoplastics.

When a particular wavelength light is incident on the nitrone compound, the nitrone is converted to an oxaziridine.

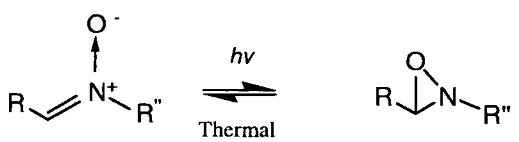


Nitrone                Oxaziridine

This oxaziridine normally has a different RI compared to the starting nitrone due to a change in its electronic structure. Thus, controlled bleaching of these nitrones with light to oxaziridine in the polymer matrix has the ability to produce structured RI changes within a polymer matrix. This structured RI change can be engineered using laser light by the means of holograms to either write data or create optical elements in polymer matrix.

As noted above, the photoreactive compound according to the present invention is a nitrone that can also be

referred to as a photochromic dye. Such photochromic dyes are capable of being written and read by electromagnetic radiation in a polymeric material. In one exemplary embodiment, the photochromic dyes can be written and read using actinic radiation i.e., from 350 to 1,100 nanometers. In a more specific embodiment, the wavelengths at which writing and reading are accomplished may be from 400 nanometers to 800 nanometers. In one exemplary embodiment, the reading and writing and is accomplished at a wavelength of 400 to 600 nanometers. In another exemplary embodiment, the writing and reading are accomplished at a wavelength of 400 to 550 nanometers. In one specific exemplary embodiment, a holographic medium is adapted for writing at a wavelength of 405 nanometers. In such a specific exemplary embodiment, reading may be conducted at a wavelength of 532 nanometers, although viewing of holograms may be conducted at other wavelengths depending on the viewing and illumination angles, and the diffraction grating spacing and angle. Examples of photochromic dyes include diarylethenes, dinitrostilbenes and nitrones.

The nitrones described herein can be optionally used in combination with other photochromic dyes. For example, diarylethenes that can be used as photoactive materials include diarylperfluorocyclopentenes, diarylmaleic anhydrides, diarylmaleimides, or a combination comprising at least one of the foregoing diarylethenes. The diarylethenes are present as open-ring or closed-ring isomers. In general, the open ring isomers of diarylethenes have absorption bands at shorter wavelengths. Upon irradiation with ultraviolet light, new absorption bands appear at longer wavelengths, which are ascribed to the closed-ring isomers. The nitrones described herein can also be used in combination with other nitrones such as α-(4-ethylamidophenyl)-N-phenylnitrone or 2,5-thiophene-bis-2-ethylhexylesterphenyl dinitrone, as well as others known in the art.

The nitrones are of generic structure (I):

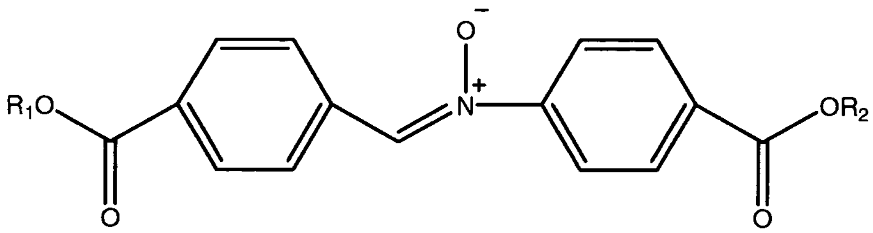

(I)

wherein $R_1$ and $R_2$ are each independently $C_1$-$C_{10}$ alkyl.

In a more specific exemplary embodiment, $R_1$ and $R_2$ are each independently $C_1$-$C_8$ alkyl, more specifically $C_1$-$C_6$ alkyl, and even more specifically $C_1$-$C_4$.alkyl In a further exemplary embodiment, $R_1$ and $R_2$ are each independently methyl or ethyl, and in a further more specific exemplary embodiment, $R_1$ is methyl and $R_2$ is ethyl. In an exemplary embodiment, the $R_1$ and $R_2$ alkyl groups are unsubstituted. In another exemplary embodiment, the $R_1$ and $R_2$ alkyl groups are substituted with known substituent groups such as hydroxyl, halogen, etc.

Exemplary compounds according to formula (I) include, but are not limited to α-(4-methoxycarbonylphenyl)-N-(4-ethoxycarbononylphenyl) nitrone, α-(4-ethoxycarbonylphenyl)-N-(4-ethoxycarbononylphenyl) nitrone, α-(4-butoxycarbonylphenyl)-N-(4-ethoxycarbononylphenyl) nitrone, α-(4-propoxycarbonylphenyl)-N-(4-pentoxycarbononylphenyl) nitrone, α-(4-hexoxycarbonylphenyl)-N-(4-butoxycarbononylphenyl) nitrone, α-(4-octoxycarbonylphenyl)-N-(4-octoxycarbononylphenyl) nitrone, and α-(4-hexoxycarbonylphenyl)-N-(4-decoxycarbononylphenyl) nitrone, α-(4-methoxycarbonylphenyl)-N-(4-methoxycarbononylphenyl) nitrone, α-(4-ethoxycarbonylphenyl)-N-(4-methoxycarbononylphenyl) nitrone, and others as can be readily envisioned from variations within the scope of formula (I).

An exemplary preparation of nitrones can include the following steps. The first step is the preparation of a hydroxylamine, as follows:

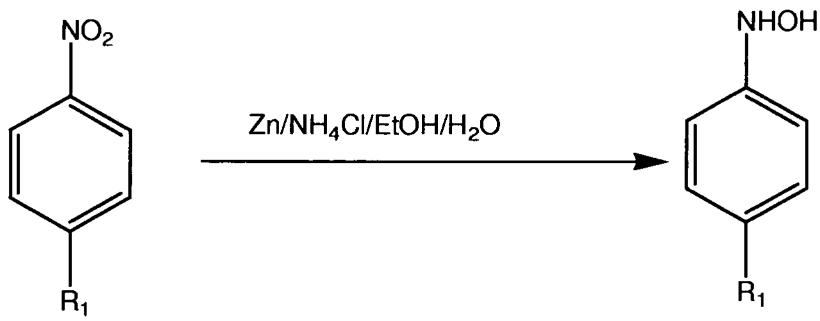


where $R_1$ = alkyl ester

Other methods are known for the preparation of hydroxylamine from a nitro compound. For example, in one method, an aromatic or aliphatic nitro compound is converted into a corresponding hydroxylamine using zinc and ammonium chloride in aqueous alcohol and then reacted with an aromatic or thiophene dicarboxaldehye. In another method (a hydrogenation method), nitro compounds can be converted to their hydroxylamine derivative using hydrogen and Pd/C or Pt/C in the presence of DMSO.

The next step is condensation of the hydroxylamine derivative with an aldehyde employing the following reaction.

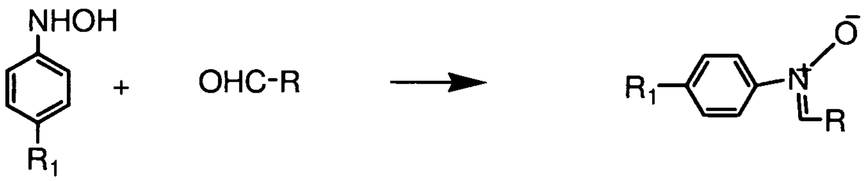

R = aromatic

Upon exposure to electromagnetic radiation, nitrones of formula (I) undergo unimolecular cyclization to an oxaziridine of formula (IA):

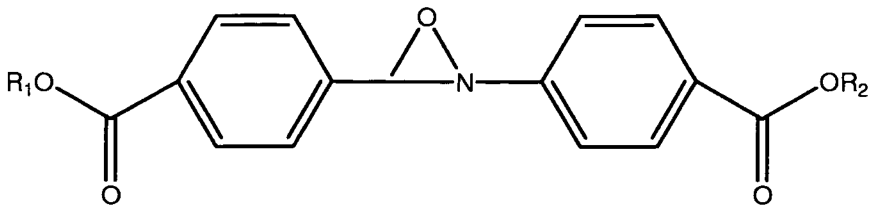

(IA)

wherein $R_1$ and $R_2$ have the same meaning as denoted above for (I).

Thus, one aspect of the invention relates to an article or photoproduct that comprises an oxaziridine compound. In some embodiments, the photocyclization of the photoreactive nitrone dye to an oxaziridine photoproduct proceeds with a high quantum efficiency, and a large refractive index change. Typically, the photocyclization is induced in only a portion of the total amount of the photoreactive nitrone dye present in a given volume element, thus providing a refractive index contrast between the unconverted dye and the oxaziridine photo-product, and providing the concentration variations of the photo-product corresponding to the holographic interference pattern, and constituting the optically readable datum.

The binder composition can include inorganic material(s), organic material(s), or a combination of inorganic material(s) with organic material(s). The binder should be an optically transparent material, e.g., a material that will not interfere with the reading or writing of the hologram. As used herein, the term "optically transparent" means that an article (e.g., layer) or a material capable of transmitting a substantial portion of incident light, wherein a substantial portion can be greater than or equal to 70% of the incident light. The optical transparency of the layer may depend on the material and the thickness of the layer. The optically transparent holographic layer may also be referred to as a holographic layer.

Exemplary organic materials include optically transparent organic polymer(s) that are elastically deformable. In one embodiment, the binder composition comprises elastomeric material(s) (e.g., those which provide compressibility to the holographic medium). Exemplary elastomeric materials include those derived from olefins, monovinyl aromatic monomers, acrylic and methacrylic acids and their ester derivatives, as well as conjugated dienes. The polymers formed from conjugated dienes can be fully or partially hydrogenated. The elastomeric materials can be in the form of homopolymers or copolymers, including random, block, radial block, graft, and core-shell copolymers. Combinations of elastomeric materials can be used.

Possible elastomeric materials include thermoplastic elastomeric polyesters (commonly known as TPE) include polyetheresters such as poly(alkylene terephthalates) (particularly poly[ethylene terephthalate] and poly[butylene terephthalate]), e.g., containing soft-block segments of poly(alkylene oxide), particularly segments of poly(ethylene oxide) and poly(butylene oxide); and polyesteramides such as those synthesized by the condensation of an aromatic diisocyanate with dicarboxylic acids and a carboxylic acid-terminated polyester or polyether prepolymer. One example of an elastomeric material is a modified graft copolymer comprising (i) an elastomeric (i.e., rubbery) polymer substrate having a glass transition temperature (Tg) less than 10° C., more specifically less than -10° C., or more specifically -200° to -80° C., and (ii) a rigid polymeric superstrate grafted to the elastomeric polymer substrate. Exemplary materials for use as the elastomeric phase include, for example, conjugated diene rubbers, for example polybutadiene and polyisoprene;

copolymers of a conjugated diene with less than 50 wt % of a copolymerizable monomer, for example a monovinylic compound such as styrene, acrylonitrile, n-butyl acrylate, or ethyl acrylate; olefin rubbers such as ethylene propylene copolymers (EPR) or ethylene-propylene-diene monomer rubbers (EPDM); ethylene-vinyl acetate rubbers; silicone rubbers; elastomeric $C_{1-8}$ alkyl(meth)acrylates; elastomeric copolymers of $C_{1-8}$ alkyl (meth)acrylates with butadiene and/or styrene; or combinations comprising at least one of the foregoing elastomers. Exemplary materials for use as the rigid phase include, for example, monovinyl aromatic monomers such as styrene and alpha-methyl styrene, and monovinylic monomers such as acrylonitrile, acrylic acid, methacrylic acid, and the $C_1$-$C_6$ esters of acrylic acid and methacrylic acid, specifically methyl methacrylate. As used herein, the term "(meth)acrylate" encompasses both acrylate and methacrylate groups.

Specific exemplary elastomer-modified graft copolymers include those formed from styrene-butadiene-styrene (SBS), styrene-butadiene rubber (SBR), styrene-ethylene-butadiene-styrene (SEBS), ABS (acrylonitrile-butadiene-styrene), acrylonitrile-ethylene-propylene-diene-styrene (AES), styrene-isoprene-styrene (SIS), methyl methacrylate-butadiene-styrene (MBS), and styrene-acrylonitrile (SAN).

Exemplary organic materials that can also be employed as the binder composition are optically transparent organic polymers. The organic polymer can be thermoplastic polymer(s), thermosetting polymer(s), or a combination comprising at least one of the foregoing polymers. The organic polymers can be oligomers, polymers, dendrimers, ionomers, copolymers such as for example, block copolymers, random copolymers, graft copolymers, star block copolymers; or the like, or a combination comprising at least one of the foregoing polymers. Exemplary thermoplastic organic polymers that can be used in the binder composition include, without limitation, polyacrylates, polymethacrylates, polyesters (e.g., cycloaliphatic polyesters, resorcinol arylate polyester, and so forth), polyolefins, polycarbonates, polystyrenes, polyamideimides, polyarylates, polyarylsulfones, polyethersulfones, polyphenylene sulfides, polysulfones, polyimides, polyetherimides, polyetherketones, polyether etherketones, polyether ketone ketones, polysiloxanes, polyurethanes, polyethers, polyether amides, polyether esters, or the like, or a combination comprising at least one of the foregoing thermoplastic polymers (either in admixture or co- or graft-polymerized), such as polycarbonate and polyester.

Exemplary polymeric binders are described herein as "transparent". Of course, this does not mean that the polymeric binder does not absorb any light of any wavelength. Exemplary polymeric binders need only be reasonably transparent in wavelengths for exposure and viewing of a holographic image so as to not unduly interfere with the formation and viewing of the image. In an exemplary embodiment, the polymer binder has an absorbance in the relevant wavelength ranges of less than 0.2. In another exemplary embodiment, the polymer binder has an absorbance in the relevant wavelength ranges of less than 0.1. In yet another exemplary embodiment, the polymer binder has an absorbance in the relevant wavelength ranges of less than 0.01. Organic polymers that are not transparent to electromagnetic radiation can also be used in the binder composition if they can be modified to become transparent. For example, polyolefins are not normally optically transparent because of the presence of large crystallites and/or spherulites. However, by copolymerizing polyolefins, they can be segregated into nanometer-sized domains that cause the copolymer to be optically transparent.

In one embodiment, the organic polymer and photochromic dye can be chemically attached. The photochromic dye can be attached to the backbone of the polymer. In another embodiment, the photochromic dye can be attached to the polymer backbone as a substituent. The chemical attachment can include covalent bonding, ionic bonding, or the like.

Examples of cycloaliphatic polyesters for use in the binder composition are those that are characterized by optical transparency, improved weatherability and low water absorption. It is also generally desirable that the cycloaliphatic polyesters have good melt compatibility with the polycarbonate resins since the polyesters can be mixed with the polycarbonate resins for use in the binder composition. Cycloaliphatic polyesters are generally prepared by reaction of a diol (e.g., straight chain or branched alkane diols, and those containing from 2 to 12 carbon atoms) with a dibasic acid or an acid derivative.

Examples of polycarbonates for use in the binder composition are those that are characterized by optical transparency, thermal stability, and good impact strength and other beneficial physical properties. Polycarbonates are generally prepared by reaction of a diol (e.g., aromatic diols like biphenol, bisphenol A, and others well-known in the art) in the presence of an appropriate catalyst, with a carbonate precursor such as phosgene (interfacial polymerization) or with a carbonate source such as diphenyl carbonate (melt polymerization).

Polyarylates that can be used in the binder composition refer to polyesters of aromatic dicarboxylic acids and bisphenols. Polyarylate copolymers include carbonate linkages in addition to the aryl ester linkages, known as polyester-carbonates. These aryl esters may be used alone or in combination with each other or more particularly in combination with bisphenol polycarbonates. These organic polymers can be prepared, for example, in solution or by melt polymerization from aromatic dicarboxylic acids or their ester forming derivatives and bisphenols and their derivatives.

Blends of organic polymers may also be used as the binder composition for the holographic devices. Specifically, organic polymer blends can include polycarbonate (PC)-poly(1,4-cyclohexane-dimethanol-1,4-cyclohexanedicarboxylate) (PCCD), PC-poly(cyclohexanedimethanol-co-ethylene terephthalate) (PETG), PC-polyethylene terephthalate (PET), PC-polybutylene terephthalate (PBT), PC-polymethylmethacrylate (PMMA), PC-PCCD-PETG, resorcinol aryl

polyester-PCCD, resorcinol aryl polyester-PETG, PC-resorcinol aryl polyester, resorcinol aryl polyester-polymethylmethacrylate (PMMA), resorcinol aryl polyester-PCCD-PETG, or the like, or a combination comprising at least one of the foregoing.

Binary blends, ternary blends and blends having more than three resins may also be used in the polymeric alloys. When a binary blend or ternary blend is used in the polymeric alloy, one of the polymeric resins in the alloy may comprise about 1 to about 99 weight percent (wt%) based on the total weight of the composition. Within this range, it is generally desirable to have the one of the polymeric resins in an amount greater than or equal to 20, preferably greater than or equal to 30 and more preferably greater than or equal to 40 wt%, based on the total weight of the composition. Also desirable within this range, is an amount of less than or equal to 90, preferably less than or equal to 80 and more preferably less than or equal to 60 wt% based on the total weight of the composition. When ternary blends of blends having more than three polymeric resins are used, the various polymeric resins may be present in any desirable weight ratio.

Exemplary thermosetting polymers that may be used in the binder composition include, without limitation, polysiloxanes, phenolics, polyurethanes, epoxies, polyesters, polyamides, polyacrylates, polymethacrylates, or the like, or a combination comprising at least one of the foregoing thermosetting polymers. In one embodiment, the organic material can be a precursor to a thermosetting polymer.

In addition to binder, the optically transparent substrate can comprise additional components such as heat stabilizers; antioxidants; light stabilizers; plasticizers; antistatic agents; mold releasing agents; additional resins; binders, blowing agents; and the like, as well as combinations of the foregoing additives.

In one embodiment, as noted above, in a method for storing holographic data, an optically transparent substrate containing the nitrone photochromic dye is irradiated with a holographic interference pattern, wherein the pattern has a first wavelength and an intensity both sufficient to convert, within a volume element of the substrate, at least some of the photoreactive dye into a photo-product, and producing within the irradiated volume element concentration variations of the photoproduct corresponding to the holographic interference pattern, thereby producing an optically readable datum corresponding to the volume element. The optically readable datum is stored in the optically transparent substrate as a hologram patterned within at least one volume element of the optically transparent substrate.

Those skilled in the art will appreciate that the lingering photosensitivity of an unconverted (residual) photochemically reactive dye can present a problem that can adversely affect the integrity of the stored data if no step is taken to stabilize the unconverted photochemically reactive dye. In the case where the unconverted photochemically reactive dye is a nitrone, protonation of the nitrone remaining following the recording of the holographic data can provide an efficient means of preventing further conversion of the nitrone to photoproducts under the influence of, for example, a read beam or ambient light.

In one embodiment, the optically transparent substrate containing the nitrone photochromic dye is irradiated with a holographic interference pattern having a first wavelength to record data. The optically transparent substrate is then irradiated with radiation having a second wavelength to stabilize the written data, and the stabilized data can then be read using radiation having a third wavelength (e.g., a "read beam"), wherein the radiation at each step can independently have a wavelength from 300 nm to 1,500 nm. In an embodiment, the first, second, and third wavelengths can be independently between 300 nm and 800 nm. In one embodiment, the first wavelength (or the writing wavelength) for writing and recording the data onto the holographic data storage medium is from 375 nm to 450 nm. In another embodiment, the first wavelength can be from 450 nm to 550 nm. In one embodiment, the first wavelength is in a range from 375 nm to 450 nm and the second wavelength is in a range from 450 to 1500 nm. In another embodiment, the first wavelength is in a range from 450 nm to 550 nm and the second wavelength is in a range from 550 to 1500 nm. In still another embodiment, the writing wavelength is such that it is shifted by 0 nm to 400 nm from the wavelength at which the recorded data is stabilized by the action of light of the second wavelength. Exemplary wavelengths at which writing and data stabilization are accomplished are 405 nanometers (writing) and 532 nanometers (stabilization). The first wavelength is also sometimes referred to as the "write" wavelength.

In one embodiment, the photochromic dye after being reacted can be converted to a non-photochromic state so that any written data cannot be destroyed. The conversion of the photochromic dye to the non-photochromic state can be induced by an electric field, by a third wavelength, by a photoacid generator or by a combination comprising at least one of the foregoing.

In another embodiment of a method of manufacturing the holographic data storage media, the photoactive material is disposed upon a first film that comprises an organic polymer. The first film behaves as a substrate upon which is disposed the photoactive material. The photoactive material can be disposed upon the first film in the form of a complete or partial layer. In yet another embodiment, a second film is disposed upon a surface of the photoactive material opposed to the surface in contact with the first film. The first and the second films can be molded or cast from solution. The second film can be disposed upon the surface of the photoactive material by molding. The photoactive material is then coated onto the surface of the first film or the surface of the second film or upon the opposing surfaces of both the first film and the second film. Examples of processes by which the photoactive material can be coated onto the surface of the film

are by brush painting, dip coating, spray painting, spin coating, or the like.

When a photochromic material is disposed upon a film to form the holographic data storage as described above, it is generally desirable to have the film having a thickness of 1 to 100,000 micrometers (μm). In one embodiment, it is desirable to have a thickness of 2 to 10,000 μm. In another embodiment, it is desirable to have a thickness of 3 to 1,000 μm. In yet another embodiment, it is desirable to have a thickness of 7 to 500 μm.

In another embodiment of a method of manufacturing the holographic data storage media, the photoactive material can be incorporated into the organic polymer in a mixing process to form a data storage composition. Following the mixing process, the data storage composition can be molded into an article that can be used as holographic data storage media. Examples of molding can include injection molding, blow molding, compression molding, vacuum forming, or the like. The injection molded article can have any geometry. Examples of suitable geometries are circular discs, square shaped plates, polygonal shapes, or the like.

The mixing processes by which the photoactive material can be incorporated into the organic polymer involves the use of shear force, extensional force, compressive force, ultrasonic energy, electromagnetic energy, thermal energy or combinations comprising at least one of the foregoing forces or forms of energy and is conducted in equipment wherein the aforementioned forces are exerted by a single screw, multiple screws, intermeshing co-rotating or counter rotating screws, non-intermeshing co-rotating or counter rotating screws, reciprocating screws, screws with pins, screws with screens, barrels with pins, rolls, rams, helical rotors, baffles, or combinations comprising at least one of the foregoing.

The mixing can be conducted in machines such as a single or multiple screw extruder, a Buss kneader, a Henschel, a helicone, an Eirich mixer, a Ross mixer, a Banbury, a roll mill, molding machines such as injection molding machines, vacuum forming machines, blow molding machine, or then like, or a combination comprising at least one of the foregoing machines.

A holographic composition containing the photochromic nitrone dye can comprise 0.1 to 50 weight percent (wt%), based on the total weight of the holographic composition. In one embodiment, the holographic composition comprises 1 to 40 wt%, based upon the total weight of the holographic composition. In another embodiment, the holographic composition comprises 2 to 20 wt%, based upon the total weight of the holographic composition. In yet another embodiment, the holographic composition comprises 3 to 10 wt%, based upon the total weight of the holographic composition.

In one embodiment, a data storage composition comprising a photoreactive nitrone and a thermoplastic polymer is injection molded to form an article that can be used for producing holographic data storage media. The injection-molded article can have any geometry. Examples of suitable geometries include circular discs, square shaped plates, polygonal shapes, or the like. The thickness of the articles can vary, from being at least 100 micrometers in an embodiment, and at least 250 micrometers in another embodiment. A thickness of at least 250 micrometers is useful in producing holographic data storage disks that are comparable to the thickness of current digital storage discs. In some embodiments, the thickness can vary from 100 micrometers to 5 centimeters. For example, for use as a DVD or CD storage device typical thickness is 600 micrometers to 1.2 millimeters.

After the molding of the data storage media the data can be stored onto the media by irradiating the media with electromagnetic energy having a first wavelength. The irradiation facilitates the conversion of the open form of the isomer to the closed form of the isomer (cyclization) of the photochromic dye thereby creating a hologram into which the data is encoded.

The holographic materials as described herein can be exposed to form holograms using any of a number of exposure setups, which are well-known in the art. A simple exposure setup, for example, is described in US 2006/0073392 A1.

<u>Examples or embodiments</u>

In one embodiment, a nitrone compound can have the formula:

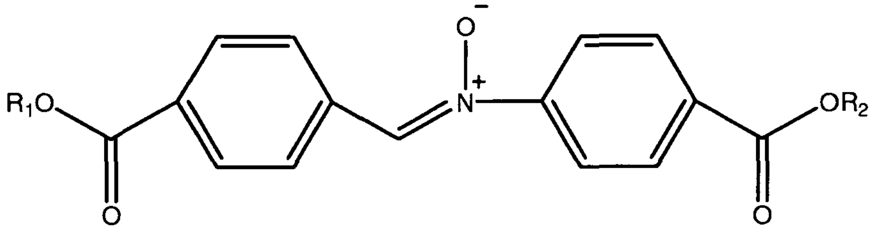

(I)

wherein $R_1$ and $R_2$ are each independently $C_1$-$C_{10}$ alkyl.

In another embodiment, a composition can comprise a polymer and a compound of formula (I), wherein $R_1$ and

$R_2$ are each independently $C_1$-$C_{10}$ alkyl.

In yet another embodiment, a holographic recording composition can comprise a polymer binder and a compound according to the formula (I), wherein $R_1$ and $R_2$ are each independently $C_1$-$C_{10}$ alkyl.

In still another embodiment, a method of manufacturing a holographic recording medium can comprise mixing a compound according to formula (I), wherein $R_1$ and $R_2$ are each independently $C_1$-$C_{10}$ alkyl, with a molten thermoplastic polymer binder to form a mixture, and forming the mixture into a holographic recording medium.

In a further embodiment, a method of recording a volume hologram can comprise exposing a holographic recording medium comprising a composition that comprises a polymer and a compound according to formula (I), wherein $R_1$ and $R_2$ are each independently $C_1$-$C_{10}$ alkyl, to mutually coherent signal and reference light sources at a wavelength that causes a change in the chemical structure of the compound of formula (I).

In the various embodiments, (i) $R_1$ and $R_2$ in formula (I) are each independently $C_1$-$C_8$ alkyl; and/or (ii) $R_1$ and $R_2$ in formula (I) are each independently $C_1$-$C_6$ alkyl; and/or (iii) $R_1$ and $R_2$ in formula (I) are each independently $C_1$-$C_4$ alkyl; and/or (iv) $R_1$ and $R_2$ are each independently methyl or ethyl; and/or (v) $R_1$ is methyl and $R_2$ is ethyl; and/or (vi) the polymer binder is a thermoplastic polymer, a thermoset polymer, or a coating; and/or (vii) the polymer binder is a polycarbonate, a polyestercarbonate or a mixture thereof; and/or (viii) the polymer binder has a glass transition temperature of at least 120°C.

The present disclosure is illustrated by the following non-limiting examples.

EXAMPLES

*Materials:*

Starting Material Ethyl-4-nitrobenzoate, 98%GC pure, CAS NO. [99-77-4] was procured from Fluka. Methyl 4-formylbenzoate 99% CAS No (1571-08-0) was from Sigma Aldrich. Basic alumina and activated carbon was from Loba Chemie.

*Synthesis:*

In a first step, the compound 4-carbethoxyphenylhydroxylamine was prepared using the following reaction:

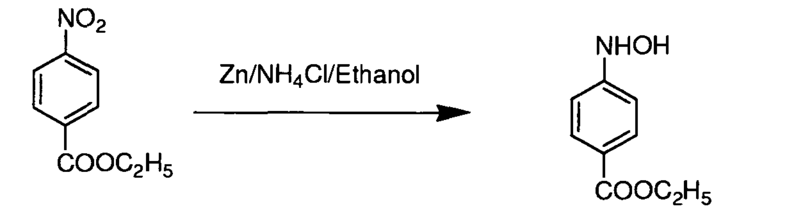


$C_9H_9NO_4$
Exact Mass: 195.05

$C_9H_{11}NO_3$
Exact Mass: 181.07

To a 2 litre 3-necked flask, 73.2 g ethyl p-nitrobenzoate, 375 ml ethanol, and 375 ml water were added. To this mixture, 26.8 g Zn and 7.16 g $NH_4Cl$ were added slowly during 30 minutes. Again to this mixture, 28.6 g Zn and 7.16 g $NH_4Cl$ were added slowly during 30 minutes. Yet again to this mixture, 28.6 g Zn and 7.16 g $NH_4Cl$ were added slowly during 30 minutes. Stirring was further continued for 30 minutes. The reaction mixture was filtered, and the precipitate cake was washed with methylene dichloride, followed by separation of the organic methylene dichloride layer of the wash solvent. The methylene dichloride wash solvent was then distilled to produce 52 ml (59 g) of an oily residue of 4-carbethoxyphenylhydroxylamine.

In a second step, α-(4-methoxycarbonylphenyl)-N-(4-ethoxycarbonylphenyl) nitrone was prepared, as follows, using the following reaction:

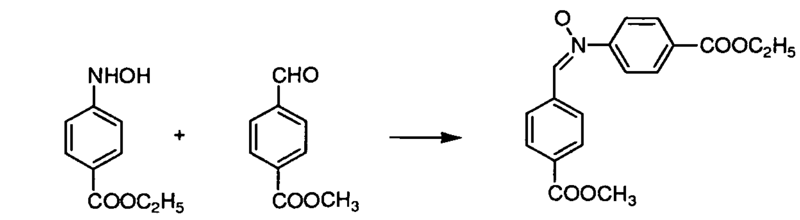

$C_9H_{11}NO_3$
Exact Mass: 181.07

$C_9H_8O_3$
Exact Mass: 164.05

$C_{18}H_{17}NO_5$
Exact Mass: 327.11

To a 2.0 liter flask were added 45.0 g methyl 4-formyl benzoate, 300 ml toluene, 50 ml acetic acid, and 57.6 g of 4-carbethoxyphenylhydroxylamine, and stirred for 10 hours. The solids were filtered out and washed with 100 ml n-hexane and 100 ml water and dried to yield 79 g of crude α-(4-methoxycarbonylphenyl)-N-(4-ethoxycarbonylphenyl) nitrone.

50 g of crude α-(4-methoxycarbonylphenyl)-N-(4-ethoxycarbonylphenyl) nitrone was dissolved with stirring in 800ml of methylene chloride. 5% basic alumina (Loba Chemie) and 5% activated carbon (Loba Chemie charcoal activated granular) were added to the above solution and stirred for 30 min at room temperature. The mixture was filtered until clear liquid was obtained. 650 ml of the clear filtrate was distilled under normal distillation with stirring, and the resulting solution was cooled to room temperature and filtered to produce a first batch of pure α-(4-methoxycarbonylphenyl)-N-(4-ethoxy carbonyl phenyl) nitrone, and a second batch was produced similarly. Finally, the remaining the remaining methylene chloride was flashed off and the residue collected to yield a total of 40 g of purified product.

<u>Analytical and Evaluative Methods:</u>

The nitrone compound of the Example above and a number of comparative compounds were subjected to analysis and evaluation as described below.

<u>Absorption Spectrum:</u>

2 mg of the compound was added to 100 ml acetonitrile. The resultant mixture was stirred for 2 hours or until complete dissolution of the dye in the acetonitrile. Spectra were recorded of each solution on a Cary/Varian 300 UV-vis spectrophotometer. Spectra were recorded in the range of 300 nanometers to 800 nanometers. Solution samples were taken in a 1 centimeter quartz cuvette and acetonitrile was taken as the blank solvent to be placed in the reference beam path for the UV-Vis measurement. The sample was exposed to an UV beam at 390 nanometers for one minute. The UV-Vis spectrum the sample was measured before and after exposure to UV light. The peak wavelength, $\lambda_{max}$, denotes the wavelength corresponding to the peak in absorption spectra and the $\lambda_{cutoff}$ corresponds to the wavelength with relative absorption of 5 percent of $\lambda_{max}$.

<u>DSC:</u>

DSC experiments were performed to study the thermal behavior of nitrones especially for melting and decomposition temperature. The melting or decomposition temperatures were measured in presence of nitrogen with a heating rate of 10°C/ min using a DSCQ10 (TA) instrument.

<u>Quantum Efficiency:</u>

<u>Preparation of Solvent Cast Samples:</u>

1 g of polycarbonate pellets was dissolved in 10 ml of methylene chloride and stirred for about 2 hours or until the polycarbonate pellets were completely dissolved in the methylene chloride. 50 mg of nitrone dye was added to the polymer solution and stirred for about 2 hours or until the nitrone was completely dissolved in the methylene chloride and polymer solution. Solvent cast samples were made by pouring the dye-polycarbonate solution inside a metal ring (5 cm radius) resting over a glass substrate. The assembly of the metal ring placed over the glass substrate was placed over a hot plate maintained at a temperature of about 40°C, and the assembly was covered with an inverted funnel to allow slow evaporation of the methylene chloride. Dried dye-doped polycarbonate films were recovered after about 4 hours. The dye-doped polycarbonate films contained 5 weight percent of the dye.

*Quantum Efficiency Measurement:*

Quantum efficiency was determined by bleaching the dried solvent cast film using a laser source. The absorption spectrum of the bleached spot was measured in situ using an Ocean Optics Spectrometer. The change in absorption at the desired wavelength was monitored over time during exposure. The absorption data is fit with a first order exponential fit to determine the time constant for bleaching. The absorption cross section is a measurement of an atom or molecule's ability to absorb light at a specified wavelength, and is measured in square cm/molecule. It is generally denoted by $\sigma(\lambda)$ and is governed by the Beer-Lambert Law for optically thin samples as shown in equation

$$\sigma(\lambda) = \ln(10) \cdot \frac{Absorbance(\lambda)}{N_o \cdot L} (cm^2).$$

*Diffraction Efficiency:*

Diffraction Efficiency is a property of the recorded hologram and hence, the holograms were recorded to measure diffraction efficiency. For recording of the hologram at 405 nm, both the reference beam and the signal beam were incident on the test sample at oblique angles of 45 degrees. The sample was positioned on a rotary stage, which was controlled by a computer. Both the reference and the signal beams had the same optical power and were polarized in the same direction (parallel to the sample surface). The beam diameters (1/e2) were 4 mm. A color filter and a small pinhole were placed in front of the detector to reduce optical noise from the background light. A fast mechanical shutter was placed in front of the laser to control the hologram recording time. In the 405 nm setup, a green 532 nm beam was used to monitor the dynamics during hologram recording. The recording power for each beam varied from 1 mW to 100 mW and the recording time varied from 10 ms to 5 s. The diffracted power from a recorded hologram was measured from a Bragg detuning curve by rotating the sample disc by 0.2 to 0.4 degrees. The reported values are not corrected for reflections off the sample surface. The power used to read the holograms was two to three orders of magnitude lower than the recording power in order to minimize hologram erasure during readout. The diffraction efficiency of the hologram, $\eta$, is calculated by using equation:

$$\eta = \frac{P_{diffracted}}{P_{reference}}.$$

where $P_{diffracted}$ is the diffracted power of the hologram.

*Sensitivity:*

Sensitivity (S) is a measure of the diffraction efficiency of a hologram recorded using a certain amount of light fluence, F. Light fluence, F, is determined as the product of light intensity (I) and exposure time (t). Sensitivity was determined using the equation

$$S = \frac{\sqrt{\eta}}{I \cdot t \cdot L} (cm/J)$$

where I is the intensity of the recording beam, *t* is the recording time, and *L* is the thickness of the holographic recording/storage medium, and $\eta$ is the diffraction efficiency.

*Results:*

The characterization data for the nitrone dye of the Example against 11 comparative nitrone dyes is compiled in the table below for comparison of optical and processing properties. The nitrone dye of the Example unexpectedly exhibited good to excellent performance across all parameters while the comparative dyes lack in at least one performance parameter.

Table I

| ID | Structure | M (nm) | Abs Max (nm) | Abs Cut off (nm) | QE | DSC T (°C) | DSC DecompT (°C) | Solubility (wt.% in $CH_2Cl_2$) | Optical Performance | | |
|---|---|---|---|---|---|---|---|---|---|---|---|
| | | | | | | | | | Blue DE | Green DE | Sensitivity (cm/J) |
| C.Ex.1 | 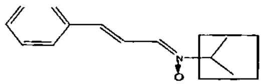 | 189 | 336 | 405 | 15 | 102 | 250 | 38 | 40 | 3.3 | 100 |
| C.Ex.2 | 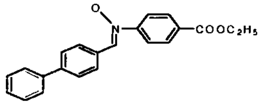 | 345 | 348 | 419 | 47 | 187 | 240 | 7.5 | 10 | 1 | 860 |
| C.Ex.3 | 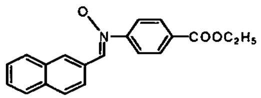 | 317 | 349 | 426 | 12 | 180 | 224 | 4.9 | 13 | 7 | 80 |
| C.Ex.4 | 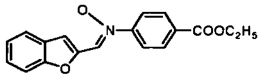 | 309 | 365 | 428 | 23 | 155 | 229 | 4 | 3 | 7 | 70 |
| C.Ex.5 | 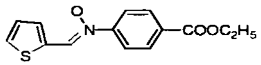 | 275 | 360 | 426 | 30 | 155 | 233 | 17.3 | 8 | 10 | 76 |
| Ex.1 | 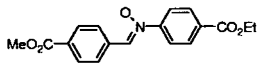 | 327 | 341 | 407 | 74 | 176 | 252 | 20 | 46 | 31 | 900 |
| C.Ex.6 | 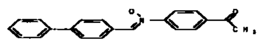 | 315 | 354 | 423 | 45 | 248 | 252 | 0.1 | 13 | 3 | 500 |
| C.Ex.7 | 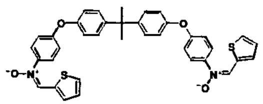 | 630 | 350 | 405 | 14 | 240 | 245 | 3 | 10 | 1 | 192 |
| C.Ex.8 | 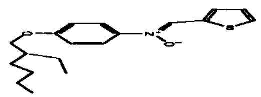 | 331 | 342 | 400 | NA | NA | 256 | 6 | 2 | 8 | |
| C.Ex.9 | 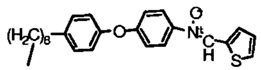 | | 363 | 430 | 17 | 111 | 210 | 5 | 7 | 3 | |

(continued)

| ID | Structure | M (nm) | Abs Max (nm) | Abs Cut off (nm) | QE | DSC T (°C) | DSC DecompT (°C) | Solubility (wt.% in $CH_2Cl_2$) | Optical Performance | | |
|---|---|---|---|---|---|---|---|---|---|---|---|
| | | | | | | | | | Blue DE | Green DE | Sensitivity (cm/J) |
| C.Ex.10 | 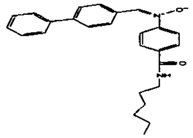 | 401 | 343 | 405 | NA | 255 | 263 | 0.3 | 9 | 10% | 2.3 |
| C.Ex.11 | 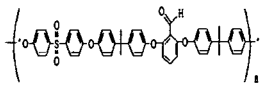 | 935 | N/A | 405 | | | 250 | 30 | 25 | 7 | 700 |

The singular forms "a," "an," and "the" include plural referents unless the context clearly dictates otherwise.

The endpoints of all ranges directed to the same component or property are inclusive and independently combinable (e.g., ranges of "less than or equal to 25 wt%, or, more specifically, 5 wt% to 20 wt%," is inclusive of the endpoints and all intermediate values of the ranges of "5 wt% to 25 wt%," etc.).

The suffix "(s)" as used herein is intended to include both the singular and the plural of the term that it modifies, thereby including at least one of that term (e.g., the colorant(s) includes at least one colorants).

"Optional" or "optionally" means that the subsequently described event or circumstance can or can not occur, and that the description includes instances where the event occurs and instances where it does not.

Unless specifically defined otherwise, technical and scientific terms used herein have the same meaning as is commonly understood by one of skill in the art to which this invention belongs. Compounds are described using standard nomenclature. For example, any position not substituted by any indicated group is understood to have its valency filled by a bond as indicated, or a hydrogen atom. A dash ("-") that is not between two letters or symbols is used to indicate a point of attachment for a substituent. For example, -CHO is attached through carbon of the carbonyl group.

The term "aromatic radical" refers to an array of atoms having a valence of at least one and comprising at least one aromatic group, i.e., a cyclic having 4n+2 "delocalized" electrons where "n" is an integer equal to 1 or greater. Aromatic radicals can include heteroatoms such as nitrogen, sulfur, selenium, silicon, and oxygen, or can be composed exclusively of carbon and hydrogen. The term "a $C_3$-$C_{10}$ aromatic radical" includes aromatic radicals having at least three but no more than 10 carbon atoms. The aromatic radical 1-imidazolyl ($C_3H_2N_2$-) represents a $C_3$ aromatic radical. Exemplary aromatic radicals include phenyl, pyridyl, furanyl, thienyl, naphthyl, phenylene, and biphenyl radicals. The aromatic radicals as that term is defined herein includes unsubstituted and substituted aromatic groups, and thus also include nonaromatic components. For example, a benzyl group is an aromatic radical, which comprises a phenyl ring (the aromatic group) and a methylene group (the nonaromatic component). The benzyl radical ($C_7H_7$-) represents a $C_7$ aromatic radical. Similarly a tetrahydronaphthyl radical is an aromatic radical comprising an aromatic group ($C_6H_3$) fused to a nonaromatic component -$(CH_2)_4$-. Substituents for the aromatic radicals include halogens, alkyl groups, alkenyl groups, alkynyl groups, haloalkyl groups, haloaromatic groups, conjugated dienyl groups, alcohol groups, ether groups, aldehyde groups, ketone groups, carboxylic acid groups, acyl groups (for example carboxylic acid derivatives such as esters and amides), amine groups, nitro groups, and the like. Exemplary substituted aromatic radicals include halogenated aromatic radicals such as 4-trifluoromethylphenyl, hexafluoroisopropylidenebis(4-phen-1-yloxy) (-OPhC$(CF_3)_2$PhO-), 4-chloromethylphen-1-yl, 3-trifluorovinyl-2-thienyl, 3-trichloromethylphen-1-yl (3-$CCl_3$Ph-), 4-(3-bromoprop-l-yl)phen-l-yl (4-$BrCH_2CH_2CH_2$Ph-), and the like; and 4-allyloxyphen-1-oxy, 4-aminophen-1-yl (4-$H_2$NPh-), 3-aminocarbonylphen-1-yl ($NH_2$COPh-), 4-benzoylphen-1-yl, dicyanomethylidenebis(4-phen-1-yloxy) (-OPhC$(CN)_2$PhO-), 3-methylphen-1-yl, methylenebis(4-phen-1-yloxy) (-OPh$CH_2$PhO-), 2-ethylphen-1-yl, phenylethenyl, 3-formyl-2-thienyl, 2-hexyl-5-furanyl, hexamethylene-1,6-bis(4-phen-1-yloxy) (-OPh$(CH_2)_6$PhO-), 4-hydroxymethylphen-1-yl (4-$HOCH_2$Ph-), 4-mercaptomethylphen-l-yl (4-$HSCH_2$Ph-), 4-methylthiophen-1-yl (4-$CH_3$SPh-), 3-methoxyphen-1-yl, 2-methoxycarbonylphen-1-yloxy (methyl salicyl), 2-nitromethylphen-1-yl (2-$NO_2CH_2$Ph), 3-trimethylsilylphen-1-yl, 4-t-butyldimethylsilylphenl-1-yl, 4-vinylphen-1-yl, vinylidenebis(phenyl), and the like.

The term "cycloaliphatic radical" refers to a radical having a valence of at least one, and comprising an array of atoms that is cyclic but not aromatic. As defined herein, cycloaliphatic radicals do not contain an aromatic group, but can include one or more noncyclic components. For example, a cyclohexylmethyl group ($C_6H_{11}CH_2$-) is a cycloaliphatic radical that includes a cyclohexyl ring (the array of atoms which is cyclic but which is not aromatic) and a methylene group (the noncyclic component). The cycloaliphatic radicals can include heteroatoms such as nitrogen, sulfur, selenium, silicon and oxygen, or can be composed exclusively of carbon and hydrogen. The term "a $C_3$ - $C_{10}$ cycloaliphatic radical" includes cycloaliphatic radicals having at least three but no more than 10 carbon atoms. The cycloaliphatic radical 2-tetrahydrofuranyl ($C_4H_7O$-) represents a $C_4$ cycloaliphatic radical. The cyclohexylmethyl radical ($C_6H_{11}CH_2$-) represents a $C_7$ cycloaliphatic radical. The aromatic radicals as that term is defined herein includes unsubstituted and substituted cycloaliphatic groups. Substituents include halogen groups, alkyl groups, alkenyl groups, alkynyl groups, haloalkyl groups, conjugated dienyl groups, alcohol groups, ether groups, aldehyde groups, ketone groups, carboxylic acid groups, acyl groups (for example carboxylic acid derivatives such as esters and amides), amine groups, nitro groups, and the like. Exemplary cycloaliphatic radicals include those comprising one or more halogen atoms, such as 2-trifluoromethylcyclohex-1-yl, 4-bromodifluoromethylcyclooct-1-yl, 2-chlorodifluoromethylcyclohex-1-yl, hexafluoroisopropylidene-2,2-bis (cyclohex-4-yl) (-$C_6H_{10}C(CF_3)_2C_6H_{10}$-), 2-chloromethylcyclohex-1-yl, 3- difluoromethylenecyclohex-1-yl, 4-trichloromethylcyclohex-1-yloxy, 4-bromodichloromethylcyclohex-1-ylthio, 2-bromoethylcyclopent-1-yl, 2-bromopropylcyclohex-1-yloxy ($CH_3CHBrCH_2C_6H_{10}O$-), and the like; and cycloaliphatic radicals include 4-allyloxycyclohex-1-yl, 4-aminocyclohex-1-yl ($H_2NC_6H_{10}$-), 4-aminocarbonylcyclopent-1-yl ($NH_2COC_5H_8$-), 4-acetyloxycyclohex-1-yl, 2,2-dicyanoisopropylidenebis(cyclohex-4-yloxy) (-$OC_6H_{10}C(CN)_2C_6H_{10}O$-), 3-methylcyclohex-1-yl, methylenebis(cyclohex-4-yloxy) (-$OC_6H_{10}CH_2C_6H_{10}O$-), 1-ethylcyclobut-1-yl, cyclopropylethenyl, 3-formyl-2-terahydrofuranyl, 2-hexyl-5-tetrahydrofuranyl, hexamethylene-1,6-bis(cyclohex-4-yloxy) (-O $C_6H_{10}(CH_2)_6C_6H_{10}O$-), 4-hydroxymethylcyclohex-1-yl (4-$HOCH_2C_6H_{10}$-), 4-mercaptomethylcyclohex-1-yl (4-$HSCH_2C_6H_{10}$-), 4-methylthiocyclohex-1-yl (4-$CH_3SC_6H_{10}$-), 4-meth-

oxycyclohex-1-yl, 2-methoxycarbonylcyclohex-1-yloxy ($2-CH_3OCOC_6H_{10}O-$), 4-nitromethylcyclohex-1-yl ($NO_2CH_2C_6H_{10}-$), 3-trimethylsilylcyclohex-1-yl, 2-t-butyldimethylsilylcyclopent-1-yl, 4-trimethoxysilylethylcyclohex-1-yl ($(CH_3O)_3SiCH_2CH_2C_6H_{10}-$), 4-vinylcyclohexen-1-yl, vinylidenebis(cyclohexyl), and the like.

The term "aliphatic radical" refers to an organic radical having a valence of at least one, a linear or branched array of atoms that is not cyclic, and at least one carbon atom. The aliphatic radicals can include heteroatoms such as nitrogen, sulfur, silicon, selenium, and oxygen or can be composed exclusively of carbon and hydrogen. A $C_1$-$C_{10}$ aliphatic radical contains at least one but no more than 10 carbon atoms. A methoxy group ($-OCH_3$) is an example of a $C_1$ aliphatic radical. An aliphatic radical, as that term is defined herein, includes unsubstituted and substituted aliphatic groups. Substitutents include halogen groups, alkyl groups, alkenyl groups, alkynyl groups, haloalkyl groups, conjugated dienyl groups, alcohol groups, ether groups, aldehyde groups, ketone groups, carboxylic acid groups, acyl groups (for example carboxylic acid derivatives such as esters and amides), amine groups, nitro groups, and the like. Exemplary substituted aliphatic radicals include halogenated aliphatic radicals such as trifluoromethyl, bromodifluoromethyl, chlorodifluoromethyl, hexafluoroisopropylidene, chloromethyl, difluorovinylidene, trichloromethyl, bromodichloromethyl, bromoethyl, 2-bromotrimethylene ($-CH_2CHBrCH_2-$), and the like; and allyl, aminocarbonyl ($-CONH_2$), carbonyl, 2,2-dicyanoisopropylidene ($-CH_2C(CN)_2CH_2-$), methyl ($-CH_3$), methylene ($-CH_2-$), ethyl, ethylene, formyl (i.e.,-CHO), hexyl, hexamethylene, hydroxymethyl (i.e., $-CH_2OH$), mercaptomethyl ($-CH_2SH$), methylthio ($-SCH_3$), methylthiomethyl ($-CH_2SCH_3$), methoxy, methoxycarbonyl ($CH_3OCO-$), nitromethyl ($-CH_2NO_2$), thiocarbonyl, trimethylsilyl ($(CH_3)_3Si-$), t-butyldimethylsilyl, 3-trimethyoxysilylpropyl ($(CH_3O)_3SiCH_2CH_2CH_2-$), vinyl, vinylidene, and the like.

"Halogens" include fluorine, chlorine, bromine, and iodine.

The term "hydrocarbyl" refers broadly to a substituent comprising carbon and hydrogen, optional with at least one heteroatoms, for example, oxygen, nitrogen, halogen, or sulfur; "alkyl" refers to a straight or branched chain monovalent hydrocarbon group; "alkylene" refers to a straight or branched chain divalent hydrocarbon group; "alkylidene" refers to a straight or branched chain divalent hydrocarbon group, with both valences on a single common carbon atom; "alkenyl" refers to a straight or branched chain monovalent hydrocarbon group having at least two carbons joined by a carbon-carbon double bond; "cycloalkyl" refers to a non-aromatic monovalent monocyclic or multicylic hydrocarbon group having at least three carbon atoms, "cycloalkenyl" refers to a non-aromatic cyclic divalent hydrocarbon group having at least three carbon atoms, with at least one degree of unsaturation; "aryl" refers to an aromatic monovalent group containing only carbon in the aromatic ring or rings; "arylene" refers to an aromatic divalent group containing only carbon in the aromatic ring or rings; "alkylaryl" refers to an aryl group that has been substituted with an alkyl group as defined above, with 4-methylphenyl being an exemplary alkylaryl group; "arylalkyl" refers to an alkyl group that has been substituted with an aryl group as defined above, with benzyl being an exemplary arylalkyl group; "acyl" refers to an alkyl group as defined above with the indicated number of carbon atoms attached through a carbonyl carbon bridge (-C(=O)-); "alkoxy" refers to an alkyl group as defined above with the indicated number of carbon atoms attached through an oxygen bridge (-O-); and "aryloxy" refers to an aryl group as defined above with the indicated number of carbon atoms attached through an oxygen bridge (-O-).

Unless otherwise indicated, each of the foregoing groups can be unsubstituted or substituted, provided that the substitution does not significantly adversely affect synthesis, stability, or use of the compound. The term "substituted" as used herein means that at least one hydrogen on the designated atom or group is replaced with another group, provided that the designated atom's normal valence is not exceeded. When the substituent is oxo (i.e., =O), then two hydrogens on the atom are replaced. Combinations of substituents and/or variables are permissible provided that the substitutions do not significantly adversely affect synthesis or use of the compound.

Exemplary groups that can be present on a "substituted" position include, but are not limited to, halogen; cyano; hydroxyl; nitro; azido; alkanoyl (such as a C2-C6 alkanoyl group such as acyl or the like); carboxamido; alkyl groups (typically having 1 to 8 carbon atoms, or 1 to 6 carbon atoms); cycloalkyl groups, alkenyl and alkynyl groups (including groups having at least one unsaturated linkages and from 2 to 8, or 2 to 6 carbon atoms); alkoxy groups having at least one oxygen linkages and from 1 to 8, or from 1 to 6 carbon atoms; aryloxy such as phenoxy; alkylthio groups including those having at least one thioether linkages and from 1 to 8 carbon atoms, or from 1 to 6 carbon atoms; alkylsulfinyl groups including those having at least one sulfinyl linkages and from 1 to 8 carbon atoms, or from 1 to 6 carbon atoms; alkylsulfonyl groups including those having at least one sulfonyl linkages and from 1 to 8 carbon atoms, or from 1 to 6 carbon atoms; aminoalkyl groups including groups having at least one N atoms and from 1 to 8, or from 1 to 6 carbon atoms; aryl having 6 or more carbons and at least one rings, (e.g., phenyl, biphenyl, naphthyl, or the like, each ring either substituted or unsubstituted aromatic); arylalkyl having 1 to 3 separate or fused rings and from 6 to 18 ring carbon atoms, with benzyl being an exemplary arylalkyl group; or arylalkoxy having 1 to 3 separate or fused rings and from 6 to 18 ring carbon atoms, with benzyloxy being an exemplary arylalkoxy group.

The term "optically transparent" as applied to an optically transparent substrate or an optically transparent plastic material means that the substrate or plastic material has an absorbance of less than 1. That is, at least 10 percent of incident light is transmitted through the material at least one wavelength in a range between about 300 nanometers and about 1500 nanometers. For example, when configured as a film having a thickness suitable for use in holographic

data storage said film exhibits an absorbance of less than 1 at least one wavelength in a range between about 300 nanometers and about 1500 nanometers.

The terms "photochemically reactive" and "photoreactive" have the same meaning and are interchangeable.

The term "volume element" means a three dimensional portion of a total volume.

The term "optically readable datum" is a datum that is stored as a hologram patterned within one or more volume elements of an optically transparent substrate.

While the invention has been described with reference to exemplary embodiments, it will be understood by those skilled in the art that various changes can be made and equivalents can be substituted for elements thereof without departing from the scope of the invention. In addition, many modifications can be made to adapt a particular situation or material to the teachings of the invention without departing from the essential scope thereof. Therefore, it is intended that the invention not be limited to the particular embodiment disclosed as the best mode contemplated for carrying out this invention.

## Claims

**1.** A nitrone compound according to the formula:

*I*

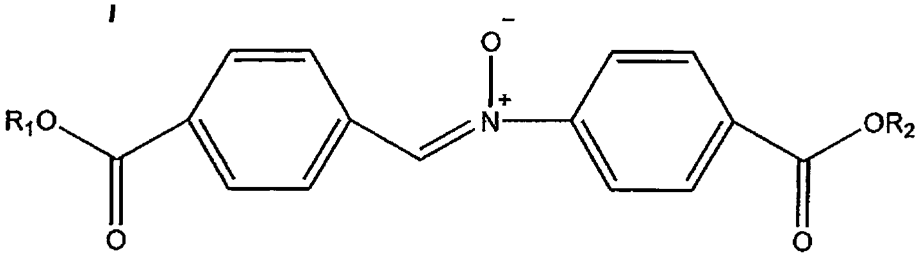

(I)

wherein $R_1$ and $R_2$ are each independently $C_1$-$C_{10}$ alkyl.

**2.** The compound of claim 1, wherein $R_1$ and $R_2$ are each independently $C_1$-$C_8$ alkyl.

**3.** The compound of claim 1, wherein $R_1$ and $R_2$ are each independently $C_1$-$C_6$ alkyl.

**4.** The compound of claim 1, wherein $R_1$ and $R_2$ are each independently $C_1$-$C_4$ alkyl.

**5.** The compound of claim 1, wherein $R_1$ and $R_2$ are each independently methyl or ethyl.

**6.** The compound of claim 1, wherein $R_1$ is methyl and $R_2$ is ethyl.

**7.** A holographic recording composition comprising the compound of any of claims 1-6 and a polymer binder.

**8.** The holographic recording composition of claim 7, wherein the polymer binder is a thermoplastic polymer, a thermoset polymer, or a coating.

**9.** The holographic recording composition of claims 7 or 8, wherein the polymer binder is a polycarbonate, a polyestercarbonate, or a mixture thereof.

**10.** The holographic recording composition of any of claims 7-9, wherein the polymer binder has a glass transition temperature of at least 120°C.

**11.** A method of manufacturing a holographic recording medium, comprising:

mixing a compound according to any of claims 1-6 with a molten thermoplastic polymer binder to form a mixture, and forming the mixture into a holographic recording medium.

**12.** The method according to claim 13, wherein the molten thermoplastic polymer binder is at a temperature of at least 120°C.

**13.** A method of recording a volume hologram, comprising exposing a holographic recording medium comprising the composition of any of claims 7-10 to mutually coherent signal and reference light sources at a wavelength that causes a change in the chemical structure of the compound of formula (I).

**Patentansprüche**

**1.** Nitronverbindung gemäß der Formel:

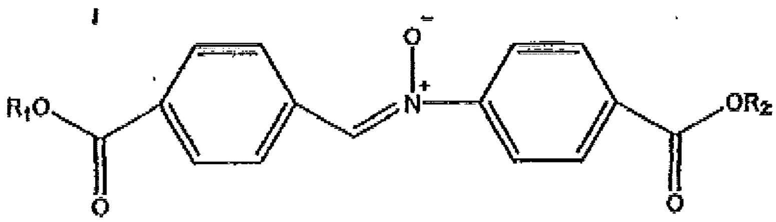

(I)

wobei $R_1$ und $R_2$ jeweils unabhängig $C_1$-$C_{10}$-Alkyl sind.

**2.** Verbindung gemäß Anspruch 1, wobei $R_1$ und $R_2$ jeweils unabhängig $C_1$-$C_8$-Alkyl sind.

**3.** Verbindung gemäß Anspruch 1, wobei $R_1$ und $R_2$ jeweils unabhängig $C_1$-$C_6$-Alkyl sind.

**4.** Verbindung gemäß Anspruch 1, wobei $R_1$ und $R_2$ jeweils unabhängig $C_1$-$C_4$-Alkyl sind.

**5.** Verbindung gemäß Anspruch 1, wobei $R_1$ und $R_2$ jeweils unabhängig Methyl oder Ethyl sind.

**6.** Verbindung gemäß Anspruch 1, wobei $R_1$ Methyl ist und $R_2$ Ethyl ist.

**7.** Holographische Aufzeichnungszusammensetzung, die die Verbindung gemäß einem der Ansprüche 1 bis 6 und ein Polymerbindemittel umfasst.

**8.** Holographische Aufzeichnungszusammensetzung gemäß Anspruch 7, wobei das Polymerbindemittel ein thermoplastisches Polymer, ein duroplastisches Polymer oder eine Beschichtung ist.

**9.** Holographische Aufzeichnungszusammensetzung gemäß Anspruch 7 oder 8, wobei das Polymerbindemittel ein Polycarbonat, ein Polyestercarbonat oder ein Gemisch davon ist.

**10.** Holographische Aufzeichnungszusammensetzung gemäß einem der Ansprüche 7 bis 9, wobei das Polymerbindemittel eine Glasübergangstemperatur von wenigstens 120 °C aufweist.

**11.** Verfahren zur Herstellung eines holographischen Aufzeichnungsmediums, umfassend:

Mischen einer Verbindung gemäß einem der Ansprüche 1 bis 6 mit einem geschmolzenen thermoplastischen Polymerbindemittel unter Bildung eines Gemischs und Verarbeiten des Gemischs zu einem holographischen Aufzeichnungsmedium.

**12.** Verfahren gemäß Anspruch 13, wobei das geschmolzene thermoplastische Polymerbindemittel eine Temperatur von wenigstens 120 °C aufweist.

**13.** Verfahren zur Aufzeichnung eines Volumenhologramms, umfassend das Belichten eines holographischen Aufzeichnungsmediums, das die Zusammensetzung gemäß einem der Ansprüche 7 bis 10 umfasst, mit einer Signal- und einer Referenzlichtquelle, die zueinander kohärent sind, bei einer Wellenlänge, die eine Veränderung der chemischen Struktur der Verbindung der Formel (I) bewirkt.

**Revendications**

1. Composé de nitrone selon la formule :

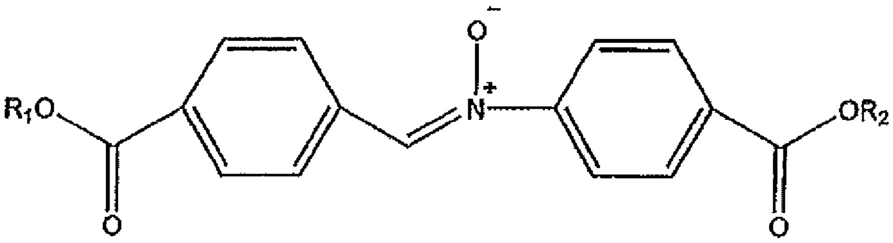

(I)

dans lequel $R_1$ et $R_2$ sont chacun indépendamment un alkyle en $C_1$ à $C_{10}$.

2. Composé selon la revendication 1, dans lequel $R_1$ et $R_2$ sont chacun indépendamment un alkyle en $C_1$ à $C_8$.

3. Composé selon la revendication 1, dans lequel $R_1$ et $R_2$ sont chacun indépendamment un alkyle en $C_1$ à $C_6$.

4. Composé selon la revendication 1, dans lequel $R_1$ et $R_2$ sont chacun indépendamment un alkyle en $C_1$ à $C_4$.

5. Composé selon la revendication 1, dans lequel $R_1$ et $R_2$ sont chacun indépendamment un méthyle ou un éthyle.

6. Composé selon la revendication 1, dans lequel $R_1$ est un méthyle et $R_2$ est un éthyle.

7. Composition d'enregistrement holographique comprenant le composé de l'une quelconque des revendications 1 à 6 et un liant polymère.

8. Composition d'enregistrement holographique selon la revendication 7, dans laquelle le liant polymère est un polymère thermoplastique, un polymère thermodurcissable ou un revêtement.

9. Composition d'enregistrement holographique selon les revendications 7 ou 8, dans laquelle le liant polymère est un poly(carbonate), un poly(estercarbonate) ou un mélange de ceux-ci.

10. Composition d'enregistrement holographique selon l'une quelconque des revendications 7 à 9, dans laquelle le liant polymère a une température de transition vitreuse d'au moins 120 °C.

11. Procédé de fabrication d'un support d'enregistrement holographique, comprenant :

    le mélange d'un composé selon l'une quelconque des revendications 1 à 6 avec un liant polymère thermoplastique fondu pour former un mélange, et la formation du mélange en un support d'enregistrement holographique.

12. Procédé selon la revendication 11, dans lequel le liant polymère thermoplastique fondu est à une température d'au moins 120 °C.

13. Procédé d'enregistrement d'un hologramme en volume, comprenant l'exposition d'un support d'enregistrement holographique comprenant la composition selon l'une quelconque des revendications 7 à 10 à des sources de signal et de lumière de référence mutuellement cohérentes à une longueur d'onde qui provoque un changement dans la structure chimique du composé de formule (I).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7824822 B2
- US 7704643 B2
- US 4996120 A
- US 5013632 A
- US 20060073392 A1
- US 20090081560 A1
- US 20090082580 A1